# EUROPEAN PATENT APPLICATION

(11) **EP 2 343 309 A1**
(43) Date of publication of application: **13.07.2011**
(21) Application number: 09818965.7
(22) Date of filing: 06.10.2009
(51) Int. Cl.: C07K 1/06, C07B 51/00, C07C 323/12

(54) **METHOD FOR PRODUCING A PEPTIDE**

(30) Priority: 07.10.2008 JP 2008260938
(71) Applicant: TOKAI UNIVERSITY EDUCATIONAL SYSTEM, Tokyo 151-0063 (JP)
(72) Inventor: HOJO, Hironobu, Hiratsuka-shi Kanagawa 259-1292 (JP); NAKAHARA, Yoshiaki, Hiratsuka-shi Kanagawa 259-1292 (JP)
(74) Representative: Polypatent
(86) International application number: PCT/JP2009/005180
(87) International publication number: WO 2010/041425

(57) **Abstract**

An object of the present invention is to provide a novel method for producing a peptide utilizing a ligation reaction in which ligation efficiency is excellent and side reactions to other functional groups in the peptide are hard to occur, in comparison with the conventional native chemical ligation methods utilizing the thiol auxiliary group. The present invention provides a method for producing a peptide which comprises a step of causing a first peptide and a second peptide to react in the presence of a reducing agent to obtain a a ligated product of the first peptide and the second peptide, wherein the first peptide contains, at the C-terminal end, an amino acid derivative having a thioester group, and the second peptide contains, at the N-terminal end, a serine or threonine derivative having a thiol auxiliary group.

SEQUENCE LISTING

<110>  Tokai University Educational System

<120>  Method for producing a peptide

<130>  P15898EP

<140>  EP 09818965.7
<141>  2009-10-06

<160>  7

<170>  PatentIn version 3.3

<210>  1
<211>  16
<212>  PRT
<213>  Artificial

<220>
<223>  synthetic peptide

<400>  1

Glu Ser Glu Glu Gly Gly Ser Asn Ala Thr Lys Lys Pro Tyr Ile Leu
1               5                   10                  15


<210>  2
<211>  10
<212>  PRT
<213>  Artificial

<220>
<223>  synthetic peptide

<400>  2

Ser Asn Ala Thr Lys Lys Pro Tyr Ile Leu
1               5                   10


<210>  3
<211>  6
<212>  PRT
<213>  Artificial

<220>
<223>  synthetic peptide

<400>  3

Glu Ser Glu Glu Gly Gly
1               5

<210>  4
<211>  4
<212>  PRT
<213>  Artificial

<220>
<223>  synthetic peptide

<400>  4

Ser Glu Glu Gly
1

<210>  5
<211>  6
<212>  PRT
<213>  Artificial

<220>
<223>  synthetic peptide

<400>  5

Lys Lys Pro Tyr Ile Leu
1               5

<210>  6
<211>  9
<212>  PRT
<213>  Artificial

<220>
<223>  synthetic peptide

<400>  6

Asn Ala Thr Lys Lys Pro Tyr Ile Leu
1               5

<210>  7
<211>  10
<212>  PRT
<213>  Artificial

<220>
<223>  synthetic peptide
Seite 2

<400>  7

Glu Val Thr Gly His Arg Trp Leu Lys Gly
1               5                   10

## Description

### Technical Field

The present invention relates to a novel method for synthesizing a peptide. More specifically, the present invention relates to a novel method for synthesizing a peptide utilizing a serene or threonine derivative having a thiol auxiliary group, and an amino acid derivative having a thioester group.

### Background Art.

As a method for chemically synthesizing a peptide, a thioester method in which peptide thioester segments prepared by a solid-phase method are repeatedly condensed (Non-Patent Document 1), and a native chemical ligation method (Non-Patent Document 2) are generally used.

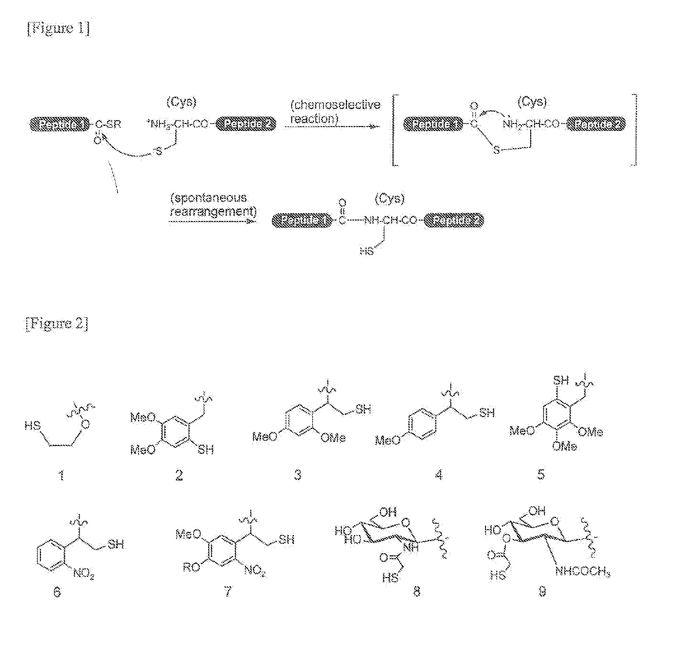

The thioester method realizes a segnient-to-segrnent condensation by activating, with silver ions.. the thioester terminal groups of the partially protected peptide thioester. On the other hand, according to the native chemical ligation method, an intermolecular thioester bond is formed between a thioester terminal group of a peptide thioester having no protecting group and a cysteine residue at an N-terminal end of another peptide, followed by formation of a peptide bond by an intermolecular aminolysis reaction of the amino group of the cysteine residue (Fig. 1).

The native chemical ligation method is more convenient than the thioester method because the peptide prepared by the solid-phase method can be used as it is in the condensation. Accordingly, the native chemical ligation method is becoming the mainstream of a chemical synthesis of a protein.

The native chemical ligation method, however, has a defect in which condensation can be performed only between peptide thioester having a thioester terminal group but no protecting group and a peptide having cysteine at the N-terminal end.

In order to overcome the defect described above, various modified thiol auxiliary groups that can be removed after ligation, have hitherto been developed by many researchers (Fig. 2). The native chemical ligation methods using the thiol auxiliary group include, for example, a method in which after ligation of cysteine is performed, the resulting cysteine is converted into alanine through a desulfurization reaction (Non-Patent Documents 3-5); a method in which after ligation is performed by using a phenylalanine residue having a thiol auxiliary group at the β-potision, the conversion into phenylalanine is performed through a desulfurization reaction (Non-Patent Document 6); a method in which after ligation of homocysteine is performed, conversion into a methionine residue is performed through methylation (Non-Patent Document 7); a method in which a thiol auxiliary group is bonded to an acetamido group or hydroxyl group of a sugar chain, and the ligation is performed, as shown in 8 or 9 of Fig. 2 (Non-Patent Documents 8-12); moreover, a method in which a thiol auxiliary group is bonded to a side-chain carboxyl group, and the ligation is performed (Non-Patent Document 13), and the like.

The conventional native chemical ligation methods utilizing the thiol auxiliary group described above, however, have various defects.

According to the methods described in Non-Patent Documents 3-5 and 6, the peptide is adsorbed on a metal, thus resulting in the lowering of a recovery rate, because of the use of a metal catalyst in the desulfurization reaction, and, furthermore, side reactions such as demethylthiolation in the methionine residue of the peptide may occur.

According to the method described in Non-Patent Document 7, other functional groups may also be methylated when the homocysteine residue is methylated.

According to the methods described in Non-Patent Documents 8-12 and 13, when the acetamido group is used, the same situations as described above result because the desulfurization reaction is finally performed, and when the sugar chain hydroxyl group is used, β-elimination of the sugar chain or racemization of α-carbon may possibly occur because an alkaline treatment is finally performed for the removal.

According to the conventional native chemical ligation methods utilizing the thiol auxiliary group, therefore, the efficiency of the ligation is low, and side reactions to the functional groups in the various amino acid residues in the peptide may possibly occur.

### Prior Art Documents

### Non-Patent Document

Non-Patent Document 1 : Hojo, H.; Aimoto, S. Bull. Chem. Soc. Jpn. 1991, 64, 111-117
Non-Patent Document 2 : Dawson P. E.; Muir, T. NV.; Clark-Lewis, I., Kent. S. B. Science 1994, 266, 776-779
Non-Patent Document 3 : Yan, L. Z.; Damson, P. E.J Am. Chem. Soc, 2001, 123, 526-533.
Non-Patent Document 4 ; Pentelute, B. L.; Kent, S. B. H. Org. Left. 2007, 9, 687-690,
Non-Patent Document 5: Wan, Q.; Dauishefsky, S. J. Angew. Chem. Int. Ed 2007, 46, 9248-9252.
Non-Parent Document 6 Crich, D.; Banerjee, A. J.Am. Chem. Soc, 2007, 129, 10064-10065. (Phe) X-Met site
Non-Patent Document 7: Saporitu, A.; Marasco, D.; Chambery A.; Botii, P.; Monti, S. M.: Pedone, C.; Ruvo, M. Biopolymers 2006, 83, 508-518.
Non-Patent Document 8, Brik, A,; Ficht, S.; Yang, Y.-Y; Bennett, C. S.; Wong, C.-H. J. Am. Chem. Soc, 2006, 128, 15026-15033.
Non-Patent Document 9: Brik, A.; Yang, Y-Y.; Ficht, S.: Wong, C.-H.J.Am. Chem. Soc, 2006. 128, 5626-5627.
Non-Patent Document 10: Yang, Y.-Y; Ficht, S.; Brik, A.; Wong, C.-H. J. Am, Chem. Soc. 2007, 129, 7690-7701.
Non-Patent Document 11: Payne, R. J.; Ficht S.; Tang. S.; Brik, A.; Yang, Y-Y.; Case, D, A,; Wong. C.-H. J. Am. Chem. Soc. 2007, 129, 13527-13536,
Non-Patent Document 12 : Ficht, S.; Payne, R. J.; Brik.. A,; Wong, C,-H. Angew. Chern. Int Ed. 2007, 46, 5975-5979.

### Side-chain auxiliary

Non-Patent Document 13: Lutsky, M.-Y; Nepomniaschiy. N.; Brik, A, Chem Commun. 2008, 1229-1231,

### SUMMARY OF THE INVENTION

### Problems to be Solved by the invention

An object to be solved by the present invention is to solve the problems of the prior art described above. That is, the object to be solved by the present invention is to provide a novel method for producing a peptide utilizing a ligation reaction in which ligation efficiency is excellent and side reactions to other functional groups in the peptide are hard to occur, in comparison with the conventional native chemical ligation methods utilizing the thiol auxiliary group. Furthermore, a second object to be solved by the present invention is to provide a compound having a novel thiol auxiliary group to be used in the production method described above.

### Means for Solving the Problem

As a result of outstanding studies for solving the problems described above, the present inventors have found that when a first peptide containing an amino acid derivative having a thioester group and a second peptide containing a serine or threonine derivative having a thiol auxiliary group are caused to react in the presence of a reducing agent, a ligated product of the first peptide and the second peptide can be obtained with high ligation efficiency and without the occurrence of side, reaction to other functional groups in the peptide; and have reached the accomplishment of the present invention.

the present invention provides a method for producing a, peptide which comprises a step of causing a first peptide and a second peptide to react in the presence of a reducing agent to obtain a ligated product of the first peptide and the second peptide, wherein the first peptide art the C-terminal end, an amino acid derivative having a thioester group, and the second peptide contains at the N-tenninal end, a serine or threonine derivative having a thiol auxiliary group,

According to a preferred embodiment of the production method of the present invention, the thiol auxiliary group is a group containing a disulfide bond.

According to a preferred embodiment of the production method of the present invention the reducing agent is a-phosphine compound,

According to a preferred embodiment of the production method of the present invention, the first peptide at the C-terminal end, an amino acid derivative having a group represented by a formula: -CO-S-R
wherein R is an alkyl group having 1 to 12 carbon atoms which may be substituted by a carboxyl group, or an aryl group having 7 to 12 carbon atoms which may be substituted by a carboxyl group; and the second peptide contains, at the N-terminal end, a serine or threonine derivative having a group represented by a formula: -R₂-S-Y₁
wherein Y₁ is a protecting group of a thiol auxiliary group, and R₂ is a methylene group which may be substituted.

Another aspect of the present invention provides a compound represented by the follonwing formula 1: 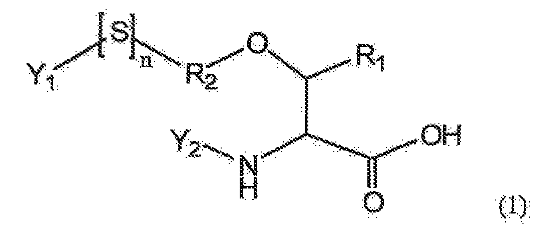 wherein Y¹ is a protecting group of a thiol auxiliary group, Y₂ is a protecting: group of an amino group, R₁ is a hydrogen atom or a methyl group, R₂ is a methylene group which may be substituted, and n is an integer of 1 to 3.

According to a preferred embodiment of the compound of the present invention. Y₁ is a t-butyl group, Y₂ is a 9-fluorenylmethoxycarbonyl group, R₁ is a hydrogen atom or a methyl group, R₂ is a methylene group, and n is 2.

### Effect of the Invention

According to the method for producing a peptide of the present invention, the thiol auxiliary group in the ligation spontaneously decomposes after the ligation reaction have proceeded, and therefore, a reaction step for removing the thiol auxiliary group is not particularly necessary. Therefore, the method for producing a peptide of the invention has a lower possibility that the side reaction occurs, and shows excellent ligation efficiency, in comparison with the conventional native chemical ligation methods utilizing the thiol auxiliary group, This usefulness of the method for producing peptide of the invention leads to an industrially highly practical method for producing a peptide in which a peptide having high stability can be easily produced in a large-scale.

### BRIEF DESCRIPTION OF THE DRAWINGS

Fig. 1 shows an outline of a native chemical ligation method;
Fig. 2 shows examples of thiol auxiliary groups, which have hitherto been used in ligation;
Fig. 3 shows an outline of a method for producing a peptide of the present invention, wherein (A) shows an assumed ligation reaction route via an intermediate A in the method for producing a peptide of the invention, and (B) is a view showing ligation of the thioester group at a C-temitnal end of a peptide in an N-termmal side, and a thiol auxiliary group at an N-tenuinal end of another peptide in a C-terminal side, in the method for producing peptide of the invention;
Fig. 4 shows a synthesis route of a serine or threonine derivative having a thiol auxiliary group;
Tig..5 shows a route of a second peptide containing a serine or threonine derivative having a thiol auxiliary group at the terminal end;
Fig. 6 shows the results of HPLC which have confirmed a synthesis of peptide 17 in Example 1 (6), wherein the chart shows the HPLC results obtained after a 24-hour ligation under condition 4 in Table 1, calculation values being as follows:
   C-terminal side chain having no thiol auxiliary group: 1427.78
   ligated product: 1997.97
   ligated product in which two N-terminal ends are ligated to a C-terminal end: 2568.16; and
Fig. 7 shows a synthesis route of a first peptide containing an amino acid derivative having a thioester group at the C-terminal end.

### EMBODIMENT FOR CARRYING OUT THE INVENTION

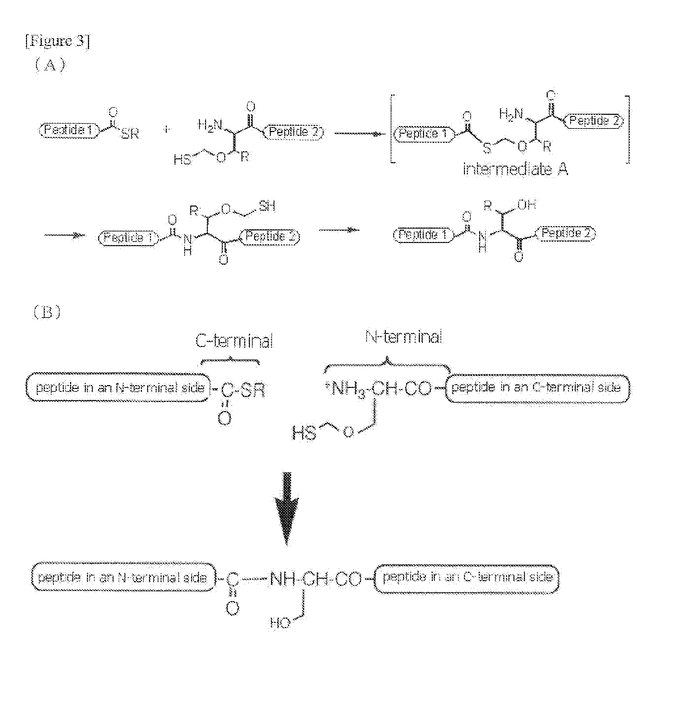

The present invention will be more specifically described below. According to the method of the invention, a first peptide containing an amino acid derivative having a thioester group at the C-terminal end and a second peptide containing a serine or threonine derivative having a thiol auxiliary group at the N-terminal end are caused to react in the presence of a reducing agent to thereby form a intermolecular thioester between the thioester group in the amino acid derivative in the first peptide and the thiol auxiliary group in the serine or threonine derivative in the second peptide; then an amido bond is formed through an intramolecular transfer; next the thiol auxiliary group is removed; and finally a ligated product of the first peptide and the second peptide is obtained (Fig. 3).

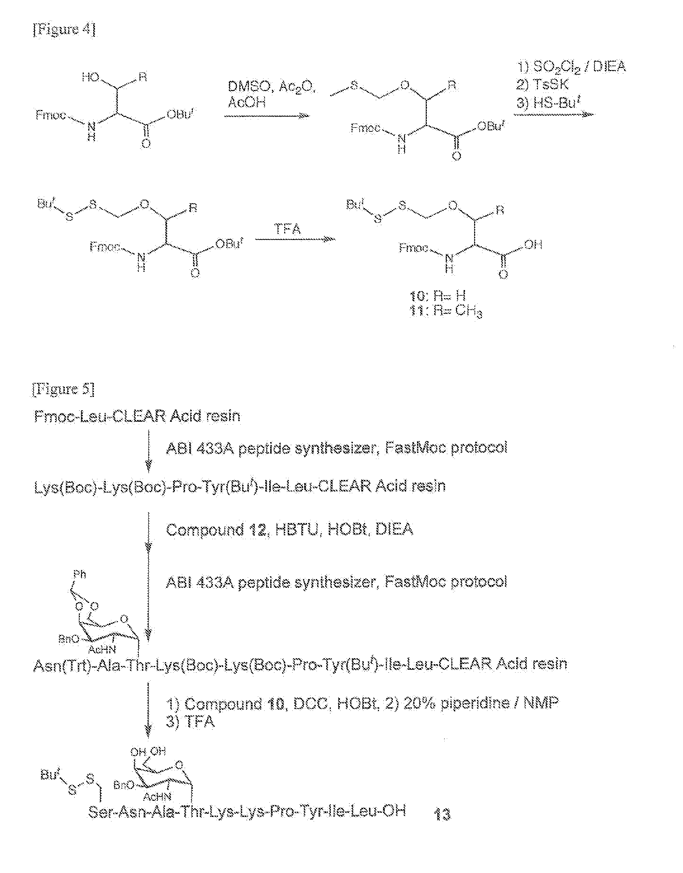

The present invention will be explained through a synthesis of a glycopeptide shown in Example described in the present specification, below. First, two kinds of amino acid derivatives (a serine derivative and a threonine derivative) having a thiol auxiliary group were synthesized according to a method described in Fig. 4. By the use of serine or threonine wherein an amino group was protected with a 9-fluorenylmethoxycarbonyl group (Fmoc) and a carboxyl group was protected with a t-butyl group (Bu^{t}) as a starting material, its side-chain hydroxyl group was methylthiomethylated with dimethyl sulfoxide (DMSO)/Ac₂O/AcOH, and then the resulting product was caused to react with potassium thiotosylate (TsSK) and t-butyl mercaptan to convert it into a disulfide. Then, the resulting converted disulfide was treated with TFA to remove the Bu^{t} ester, whereby a serine or threonine derivative having the following formula 2: 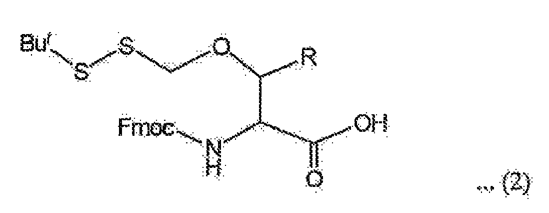 wherein R is a hydrogen atom or a methyl group was obtained.

By utilizing the serine derivative or threonine derivative obtained as above, a synthesis of a glycopeptide contulakin-G (pGlu-Ser-Glu-Glu-Gly-Gly-Ser-Asn-Ala-Thr(GalNAc)-Lys-Lys-Pro-Tyr-ile-Leu-OH) was attempted. Meanwhile although a nature contulakin-G has Gal-GalNAc as a sugar chain which bonds to Thr residue at the 10-position, a synthesis of a contalakin-G haying a monosaccharide which bonds to Thr residue at the 10-position was attempted here, in order to confirm the progress of the reaction according to the present invention.

When the glycopeptide is synthesized, first, a synthesis of a C-terminal-side peptide containing, at the N-terminal end, a serine derivative having a thiol auxiliary group, and an N-terminal-side-peptide containing, at the C-terminal end, an amino acid derivative having a thioester group was attempt, which are two part of the glycopeptide chain described above divided between the Gly residue at the 6-position and the Ser residue at the 7-position.

According to a method described in Fig. 5, the peptide chain length having the C-terminal-side peptide was extended by using an automatic synthesizer according to an Fmoc method, using an Fmuc-Leu-CLEAR Acid resin as a starting material. Into the extended peptide chain was introduced compound 12 having the following formula 12: 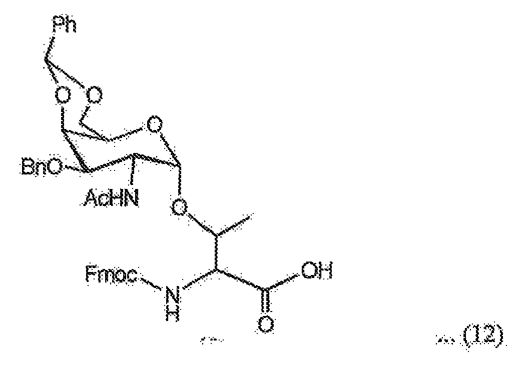 which was synthesized by using HBTU (2-(1H-Benzotriazole-1-yl)-1,1,3,3-tetramethyluronium hexafluorophosphate)-HOBt (Hydroxybenzotriazole) according to a conventional method, and then the peptide chain length was further extended, into which the serine derivative having the thiol auxiliary group was introduced by utilizing an N,N'-dicyclohexylcarbodiimide (DCC)-HOBt method. The resulting peptide chain was treated with TEA, and the peptide was eliminated from the resin, followed by deprotection. Purification by using a reversed phase HPLC was made to obtain peptide. 13 having, at the N-terminal end, the serine derivative having the thiol auxiliary group, represented by the following formula 13:

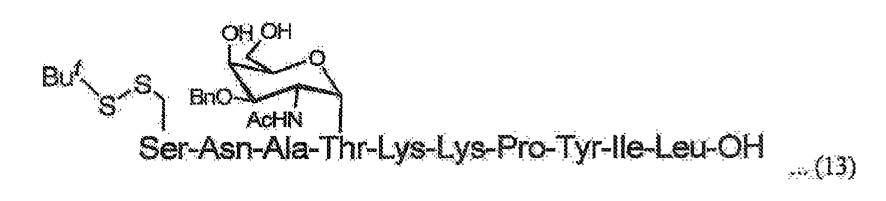

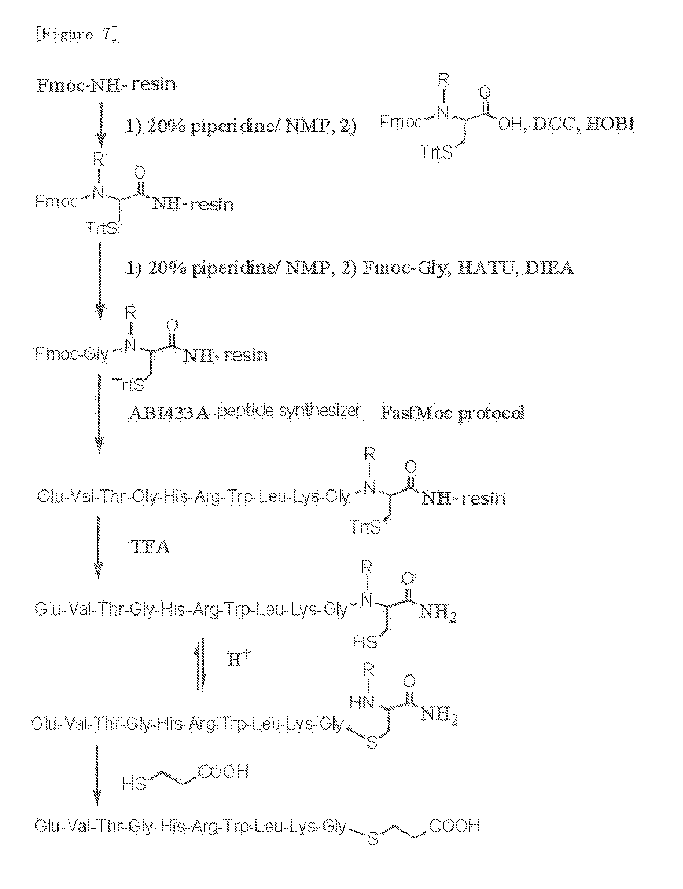

Peptide 14 (pGlu-Ser-Glu-Glu-Gly-Gly-SC₆CH₄COOH), a peptide containing, at the C-terminal end, an amino acid derivative having a thioester group, was synthesized with reference to a method described in WO 2008/044628 (Fig. 7).

The two kind peptides, peptide 13 and peptide 14, obtained as above, were condensed according to a ligation method under various conditions shown in Table 1.

**Table 1**

| Reaction Condition ^{a)} | Yield ^{b)} |
|---|---|
| 1: 0.1 M phosphate buffer solution (including 0.2 M MPAA) | 0% |
| 2:0.1 M phosphate buffer solution (including 7.5 mM TCEP) | 25% |
| 3: 0.2 M phosphate buffer solution (including 15 mM TCEP): CH₃CN (1:1) | 39% |
| 4: 0.1 M phosphate buffer solution (including 15 mM TCEP): CH₃CN(1:1) ^{c)} | 49% |

a) In each case, the reaction was performed at pH 7.0 for 24 hours. b) The yield is obtained by dividing a peak area of a desired product at 280 nm by the total of the peak area of desired product, a peak area of an unreacted C-terminal segment, and a peak area of a by-product derived from the C-terminal end. c) A reaction liquid is 10-fold diluted with DMF 10 minutes after the reaction,

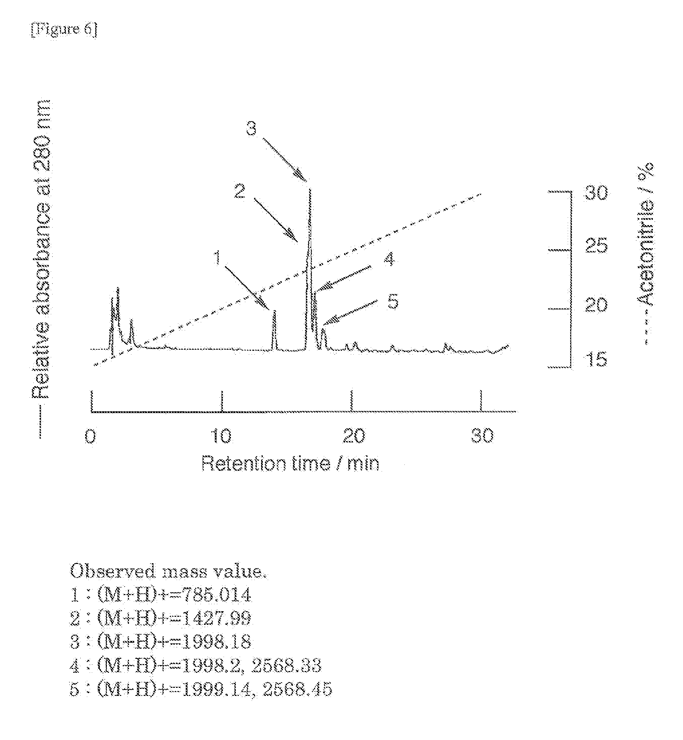

First, ligation was attempted by dissolving peptides 13 and 14 in 0.1 M phosphate buffer solution (pH 7.0) in a molar ratio of 1:1, and adding 0,2 M mercaptophenyl acetic acid (MPAA) thereto (condition 1 in Table 1). It was expected that the mercaptophenyl acetic acid would inhibit the hydrolysis of the thioester bond in peptide 14, and would have an action of reducing the thiol auxiliary group in peptide 13 having been protected with the disulfide bond, to start the ligation, under this condition. However, the ligation did not occur at all, and only the C-terminal peptide from which the thiol auxiliary group had been removed and the N-terminal thioester, and their hydrolyzates were able to be obtained. From this result, it was expected that the thiol auxiliary group quickly decomposed just after it was reduced to a fire thiol group, and that the intramolecular aminolysis, which occurred after the thioester bond was formed between the two peptides, proceeded very slowly in comparison with a case of utilizing Cys residue, and a thioester exchange occurred quickly with the mercaptophenyl acetic acid added. Therefore, in order to inhibit the tioester exchange with the thiol added, the ligation was attempted in the absence of the thiol under conditions 2 to 4 subsequently described. In addition, in order to reduce the thiol auxiliary group into a state in which a free thiol group was present, the addition of a reducing agent, tris(2-carboxyethyl)phosphine (TCEP) to the solution was attempted. First, when the ligation was performed under condition 2 in which only phosphate buffer including TCEP is used, the yield of glycopeptide 17 (pGlu-Ser-Glu-Glu-Gly-Gly-Ser-Asn-Ala-Thr(GalNAcBn)-Lys-Lys-Pro-Tyr-Ile-Leu-OH) was 25%. Therefore, in order to inhibit the hydrolysis after the thioester bond is formed between the peptides, the ligation was performed under a condition in which 50% of acetonitrile was added, as shown in the condition 3 in Table 1, whereby the yield was improved up to 39%. Further, since the intermolecular thioester was formed quickly in about 10 minutes, the intramolecular aminolysis was performed preferentially under condition 4 in which the reaction solution was 10-fold diluted with dimethyl formamide 10 minutes after the reaction, whereby the yield was improved up to 49% (Fig. 6).

From the results described above, it was found that the method for producing a peptide of the present invention does not have the concern for a side reaction during a removal reaction after the ligation, because such a removal reaction is not necessary, and is a highly practical method capable of performing efficient ligation. In addition, the method for producing a peptide of the invention is a method in which further improvement of ligation yield can be expected by using the thiol auxiliary group having high stability.

The method for producing a peptide of the invention contains the step of causing the first peptide to react with the second peptide in the presence of the reducing agent to give the ligated product of the first peptide and the second peptide (hereinafter sometimes may be referred to as a "ligation step"). The ligation step will be explained bellow.

The frist peptide that is a starting material in the ligation step has an amino acid derivative with a thioester group at the C-terminal end. The second peptide that is another starting material in the ligation step has a serine derivative with a thiol auxiliary group or a threonine derivative with a thiol auxiliary group at the N-terminal end.

The thiol auxiliary group in the serine or threonine derivative in the second peptide reacts with the thioester group in the amino acid derivative in the first peptide in the presence of a reducing agent to form a thioester condensate that is an intermediate. In the formed thioester condensate, an S → N intramolecular transition spontaneously occurs, followed by the removal of the thiol auxiliary group. Accordingly, the thiol auxiliary group in the serine or threonine derivative, which is present at the N-terminal end of the second peptide, is not particularly limited, as long as it can react with the thioester group in the amino acid derivative, which is present at the C-terminal end of the first peptide. For example, groups: Y₁-(S)ₙ-R₂- wherein Y₁ is a protecting group of the thiol auxiliary group, R₂ is a methylene group, which may be substituted, and n is an integer of 1 to 3 are preferable, and groups: Y₁-(S)₂-R₂- wherein Y₁ and R₂, are as defined above are more preferable. The group R₂ is preferably a methylene group, more preferably a methylene group in which the hydrogen atom is substituted by another atom or group, and further more preferably a methylene group in which the hydrogen atom is substituted by an electron-withdrawing atom or group. Examples of R₂ include -CH₂-, -CH(CF₃)-, -CH(F)-, and the like. The thioester group in the amino acid derivative, which is present at the C-terminal end of the first, peptide, is not particularly limited as long as it is caused to react with the thiol auxiliary group in the serine or threonine derivative, which is present at the N-terminal end of the second peptide. For example, groups: -CO-S-R wherein R is an alkyl group having 1 to 12 carbon atoms, which may be substituted by a carboxyl group, or an aryl group having 7 to 12 carbon atoms, which may be substituted by a carboxyl group are preferable, Examples of the alkyl group having 1 to 12 carbon atoms in R include a methyl group, an ethyl group, a propyl group, an isopropyl group, a butyl group, an isobutyl group, a pentyl group, an isopentyl group, a hexyl group, a heptyl group, and the like. Examples of the aryl group having 7 to 12 carbon atoms include a phenyl group, a benzyl group, a tolyl group, a xylyl group, a mesityl group, a cumenyl group, a phenethyl group, a naphthyl group, and the like. The hydrogen atom is the alkyl group having 1 to 12 carbon atoms and the aryl group having 7 to 12 carbon atoms in R described above may be substituted by a carboxyl group. A preferable example of R is -C₆H₄CH₂COOH.

The serine or threonine derivative is introduced into the peptide chain in the form of a compound, for example, represented by the following formula 1: 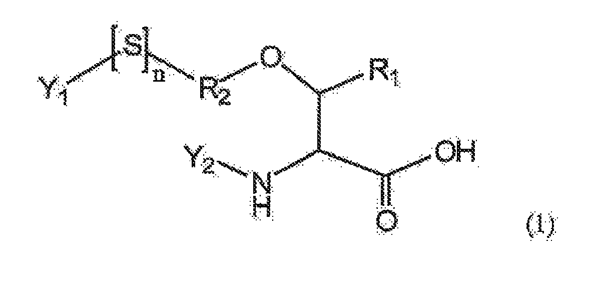 wherein Y₁ is a protecting group of the thiol auxiliary group, Y₂ is a protecting group of the amino group, R₁ is a hydrogen atom or a methyl group, R₂ is a methylene group which may be substituted, and n is an integer of 1 to 3.

Examples of Y₁ that is the protecting group of the thiol auxiliary group and Y₂ that is the protecting group of the amino group in the serine or threonine derivative each include a t-butyl group, a 9-fluorenylmothoxycarbonyl group, a trityl group, an acetamidomethyl group, a benzyl group, a 4-methylbenzyl group, a 4-methoxybenzyl group, a 3-nitro-2-pyridinesulfenyl group, an ethylmercapto group, a tert-butylmercapto group, and the like, Y₁ being different from Y₂, Y₁ is preferably t-butyl group, and Y₂ is preferably 9-fluorenylmethoxycarbonyl group.

In the serine or threonine derivative. R₂ is preferably a methylene group, more preferably a methylene group in which the hydrogen atom is substituted by another atom or group, and further more preferably a methylene group in which the hydrogen atom is substituted by an electron-withdrawing atom or group. Examples of R₂ include -CH₂-, -CH(CF₃)-, -CH(F)-, and the like. In order to improve the stability of the thiol auxiliary group and increase the ligation yield, substituted methylene groups such as -CH(F)- and -CH(CF₃)- are preferable.

In the serine or threonine derivative, n showing the number of sulfur atoms is preferably within a range of an integer of 1 to 3, more preferably 1 or 2, and further more preferably 2.

The synthesis of the serine or threonine derivative is shown bellow (Fig. 8)

First, serine or threonine in which the amino group is protected with Fmoc and the carboxyl group is protected with But is caused to react with a sulfur-containing compound such as dimethyl sulfoxide (DMSO), methyl ethyl sulfoxide or diethyl sulfoxide, and Ac₂O and AcOH, whereby the side-chain hydroxyl group is alkylthioalkylated. The reaction can be performed at an appropriate temperature (for example, 10 to 60°C) for a predetermined time. The alkylthioalkylated product can be recovered as, for example, a precipitate. Furthermore, the resulting precipitate can also be dissolved in as appropriate solvent such as ethyl acetate followed by sequential washing with an aqueous saturated sodium bicarbonate solution and an aqueous saturated sodium chloride solution, and the like. The resulting alkylthioalkylated product is subjected to appropriate treatments such as drying and concentration, followed by separation and purification. For the separation and purification, it is preferable to use, for example, a silica-gel chromatography.

Next, when a converted disulfide is obtained from the alkylthioalkylated product, the following procedures are performed,

The separated and purified alkylthioalkylated product is dissolved in a basic solvent containing a neutralizing agent, preferably N,N-diisopropylethylamine (DIEA) at an appropriate temperature (for example, -10 to 10°C) for a predetermined time to neutralize it, then the resultant product is mixed with a solvent containing a halogenating agent, preferably sulfuryl chloride at an appropriate temperature (for example, 10 to 60°C) for a predetermined time to halogenate the alkylthioalkyl group, and after that the resulting product is mixed with a solvent containing an agent that converts to a thiol groups preferably potassium thiotosylate at an appropriate temperature (for example, 10 to 60°C) for a predetermined time to substitute thiol for the halogen. To the resulting produce is added Bu^{t}-SH, and the mixture is stirred at an appropriate temperature (for example, 10 to 60°C) for a predetermined time, which is diluted with a proper organic solvent to obtain a converted disulfide. An organic layer containing the obtained converted disulfide may also be washed with a proper aqueous solvent (for example an aqueous saturated sodium bicarbonate solution, an aqueous sodium chloride solution, and the like The converted disulfide is subjected to proper procedures such as drying and concentrate followed by separation and purification. For the separation and purification, it is preferable to use, for example, a silica-gel chromatography, A converted trisulfide can be obtained from the alkylthioalkylated product in the same manner as above.

The converted disulfide is dissolved in TFA, preferably TFA containing thioanisole at an appropriate temperature (for example, 10 to 60°C) for a predetermined time. The thioanisole in the TFA can trap Bu¹ cations, which may be derived from a decomposition of Bu^{t} ester. The obtained serine or threonine derivative is subjected to proper procedures such as drying and concentration, followed by separation and purification, For the separation and purification, it is preferable to use, for example, a silica-gel chromatography.

Among of examples of the serine or threonine derivative, compounds of the above-mentioned formula 1 wherein Y₁ is a t-butyl groups, Y₂ is 9-fluorenylmethoxycarbonyl group, R₁ is a hydrogen atom or a methyl group, R₂ is a methylene group, and n is 2 are preferable.

The second peptide can be synthesized by, for example, bonding the serine or threonine derivative to the N-terminal end of the peptide chain which has been extended by using a peptide automatic synthesizer. For example, an amino acid residue bonded to a resin is used as a starting material, and the peptide chain length is extended using an automatic synthesizer according to an Fmoc method. Then, the serine or threonine derivative (for example, Fmoc-Ser(CH₂-S-S-Bu*^{t}*)-OH) is added thereto, whereby it is condensed to the N-terminal end of the resin-bonded peptide. After that, the terminal Fmoc in the obtained resin is removed, dried it, a mixed liquid of TFA phenol - distilled water : thioanisole is added thereto, and the mixture is stirred. The liquid is removed by passing nitrogen gas, to which diethyl ether is added to precipitate a peptide. After the precipitate is dried under vacuum, the peptide is extracted with an aqueous acetonitrile solution, and the resin is filtered, Purification with a reversed phase HPLC can provide a second peptide. The synthesis reaction of the second peptide may be performed at a proper temperature (for example, 10 to 60°C) for a predetermined time. In the extension of the peptide chain, an amino acid residue modified with sugar or the like may be introduced in a conventional technique. For example, a resin whose peptide chain length is extended, and a sugar-modified amino acid protected with Fmoc are added to a solution of HBTU-HOBt / DMF and DIEA in N-methylpyrrolidone (NMP), and the reaction is performed at a proper temperature (for example, 50°C) for a predetermined time, whereby the sugar-modified amino acid residue can be added on the peptide chain, Examples of the thus obtained second peptide may include peptide 13 described above.

The synthesis of the first peptide is not particularly limited as long as a peptide containing, at the C-termmal end, an amino acid derivative having a thioester group can be obtained. The synthesis can be performed according to, for example, a direct synthesis method using a Boc method (Hojo. H.; Aimoto, S. Bull. Chem. Soc. Jpn., 64, 111- 117, (1991)); a direct synthesis method using an Fmoc method (Li, X.; Kawakami, T.; Aimoto, S. Tetrahedron Lett., 39, 8669-8672, (1998)); a method in which a peptide chain is constituted using an Fmoc method and then the C-terminal end is thioesterified (Shin, Y,; Winans, K. A,; Backes, B. J,; Kent, S. B. H., Ellman, J. A.; Bertozzi, C. R. J. Am. Chem. Soc., 121, 11684-11689, (1999)); or the like. In addition, the synthesis can also be performed in a method described in International Patent Application No. PCT / JP2007 / 069555. A method described in WO2008/044628 will be explained below.

According to the method described in WO2008/044628, a peptide containing at the C-terminal side, an amino acid derivative having a thioester group can be synthesized through following steps (i) to (iv).

In step (i), the deprotection of the Fmoc group is performed by, for example, treating an Fmoc-CLEAR amide resin with piperidine/N-methylpyrrolidone (NMP), and the resulting resin is caused to react with Fmoc-N(R)-CH(CH2SY)-C(=O)OH in HOBt/NMP and N,N'-dicyclohexyl carbodiimide (DCC)/NMP. The reaction may be performed at an appropriate temperature (for example. 40 to 60°C).

The group R in Fmoc-N(R)-CH(CH₂SY)-C(=O)OH is an alkyl group having 1. to 12 carbon atoms or an aralkyl group having 7 to 12 carbon atoms. Examples of the alkyl group can include, a methyl group, an ethyl group, a propyl group, a butyl group, a pentyl group, a hexyl group, and the like, which may be linear, branched or cyclic hydrocarbon groups. Examples of the aralkyl group can include a benzyl group, and the like, R is preferably a methyl group, an ethyl group, an isobutyl group or a benzyl group.

Step (ii) is a step in which after the deprotection of the Fmoc group in the Fmoc-(amino acid residue)ₙ-N(R)-CH(CH₂SY)-C(=O)NH-resin in which n is 0 or 1, obtained in step (i), is performed, the resulting product is caused to react with an Fmoc-amino acid, if necessary, to produce an Fmoc-(amino acid residue)ₙ-N(R)-CH(CH₂SY)-C(=O)NH-resin, in which n is 1 or 2, and then the deprotection of the Fmoc group and the reaction with the Fmoc-amino acid are repeated to produce an X-N(R)-CH(CH₂SY)-C(=O)-NH-resin. When in step (i), the Fmoc-(amino acid residue)ₙ-N(R)-CH(CH₂SY)-C(=O)NH-resin is produced by causing the Fmoc-(amino acid residue)ₙ₋N(R)-CH(CH₂SY)-C(=O)OH (n is 1) to react, the Fmoc-amino acid may be caused to react or not in step (ii),

In step (ii), specifically, the reaction product from step (i) is washed with NMP, and then the resin is shaken in Ac₂O-DIEA/NMP for 5 minutes. After washing with NMP and the removal of the Fmoc group with piperidine/NMP are performed, a solution of Fmoc-Gly and hexafluorophosphate, O-(7-azabenzotriazole-1-yl)-N,N,N',N'-tetramethyluronium (HATU), and N,N-diisopropylethylamine (DIEA) dissolved in NMP is added thereto, and the reaction is performed. The reaction may be performed at an appropriate temperate (for example, 40 to 60°C) for a predetermined time. Next, after the reaction product is washed with NMP, the dehydration of the Fmoc group and reaction with the Fmoc-amino acid are repeated by using a conventional peptide synthesizer in accordance with, for example a Fast Moe method, whereby the peptide chain length can be extended.

Step (iii) is a step in which the X-N(-R)-CH(CH₂SY)-C(=O)-NH-resin obtained in step (ii) is caused to react with a deprotecting agent (for example, trifluoroacetic acid, and the like), whereby the elimination from the resin and deprotection of the thiol group are performed. Specifically, Reagent K is added to the product (resin) in step (ii), and the reaction can be performed at room temperature. Here, Reagent K refers to a reagent of TEA: H₂O: thioanisole: ethanedithiol; phenol = 82.5 : 5 : 5 : 2.5 : 5.

Step (iv) is a step in which the compound obtained in step (iii) is caused to react with an acidic thiol to produce a thioester compound. Examples of the acidic thiol to be used here may include mercaptocarboxylic acid and a mixture of mercaptan and a carboxylic acid, HSCH₂CH₂COOH(MPA), HSC₆H₄CH₂COOH(MPAA), or a mixture of thiophenol and acetic acid can be more preferably used.

In step (iv), TFA is removed from the product from step (iii) through nitrogen stream, to which an ether is added to generate a precipitate. The precipitate is washed with an ether, and then is dried. The crude peptide is extracted with an aqueous acetonitrile solution containing TFA, which is diluted with an aqueous solution of acetonitrile of acidic thiol such as an aqueous solution of 3-mercaptopropionic acid or 4-mercaptophenylacetic acid, and the resulting product its allowed to stand for several hours to several ten hours, whereby a peptide thioester compound represented by X-S-CH₂CH₂-COOH or X-S-C₆H₄CH₂COOH can be obtained.

Examples of the first peptide obtained through steeps (i) to (iv) can include peptide 14 described above.

In addition, a compound represented by Z-N(R)-CH(CH₂SY)-C(=O)OH wherein Z is a hydrogen atom or a 9-fluorerylmethioxycarbanyl group, R is an alkyl group having 1 to 12 carbon atoms or an aralkyl group having 7 to 12 carbon atoms, and Y is a protecting group of thiol R and Y being described in detail above, is synthesized at follows:
First, a compound represented by YSCH₂CH(NH₂)C(=O)OH wherein Y is a protecting group of thiol group or cysteine in which the thiol groups is protected, and a compound represented by R¹CHO wherein R¹ is a hydrogen atom, an alkyl group having 1 to 11 carbon atoms or an aryl group having 6 to 11 carbon atoms are caused to react to produce a compound represented by YSCH₂CH(N=CHR¹)C(=O)OH wherein Y and R¹ are as defined above. Here, the alkyl group in R¹ is preferably an alkyl group having 1 to 9 carbon atoms, more preferably an alkyl group having I to 6 carbon atoms. Examples thereof may include a methyl group, an ethyl group, a propyl group, a butyl groups a pentyl group, a hexyl groups and the like, which may be any of linear, branched or cyclic hyrdocarbons. Particularly preferable R¹ₛ are a methyl group, an isopropyl group and a phenol group. The reaction thereof with the compound reprinted by R¹CHO can be performed by dissolving the cysteine in which the thiol group is protected in water containing ethanol and potassium hydroxide, adding the compound represented by R¹CHO thereto, and stirring the mixture at an appropriate temperature (for example, room temperature) for a predetermined time.

Subsequently, the compound represented by YSCH₂CH(N=CHR¹)C(=O)OH wherein Yank R¹ are as defined above is caused to react with a hydrogenating agent (for example, NaBH₄ or NaBH₃CN) to produce a compound represented by YSCH₂CH(NHC₂R¹)C(=O)OH wherein Y and R¹ are as defined above, and, if necessary, the resulting product is caused to react with 9-fluorenylmethoxycarbon-N-hydroxysuccinimide ester (hereinafter " Fmoc-OSu") to protect the amino group with Fmoc group, whereby a compound represented by Z-N(R)-CH(CR₂SY)-C(-O)OH wherein Z is a hydrogen atom or a 9-fluorenylmethoxycarbonyl group, R is a alkyl group having 1 to 12 carbon atoms, or an aralkyl group having 7 to 12 carbon atoms, and Y is a protecting group of thiol group can be produced, The hydrogenating agent may be caused to react in the presence of a base such as sodium hydroxide. Specifically, the hydrogenating agent (for example, NaBH₄ or NaBH₃CN) is added to the compound represented hy YSCH₂CH(N-CHR¹)C(=O)OH, and the mixture is stirred at a proper temperature, whereby the reaction can be performed. When NaBH₄ is used, it is preferable to use it as a solution thereof dissolved in an aqueous sodium hydroxide solution; whereas when NaBH₃CN is used, a base is not particularly necessary. Furthermore, when the amino group is protected with the Fmoc group by causing it to react with Fmoc-OSu, the amino group can be protected with the Fmoc group by causing it to react with the Fmoc group in the presence of sodium carbonate in a proper solvent (for example, 1,2-dimethoxyethane).

The ligation reaction of the first peptide and the second peptide can be performed in the presence of a reducing agent in a buffer solution or a mixed solution of a buffer and an organic solvent.

The reducing agent is not particularly limited, as long as it can reduce the thiol auxiliary group in the serine or threonine derivative to a state having a free thiol group. It is preferable, to use, for example, a water-soluble phosphine such as triscarboxyethyl phosphine (TCEP) or trishydroxymethyl phosphine, trimethyl phosphine, triethyl phosphine, triisopropyl phosphine, tributyl phosphine, triphenyl phosphine, or the like; it is more preferable to use water-soluble phosphine, further more preferable to use TCEP. When a thiol such as thiophenol, mercaptophenylacetic acid, t-butyl mercaptan or dithiothreitol is used as a reducing agent, the ligation efficiency may highly likely lowers, because of occurrence of thioester exchange. The concentration of the reducing agent can be suitably adjusted depending to the kind of the educing agent, as long as it can reduce the thiol auxiliary group in the serine or threonine derivative to a state having the free thiol group. For example, when TCEP is used was the reducing agent, the concentration is preferably from 7.5 to 50 mM, more preferably from 15 to 30 mM.

The buffer solution to be used in the ligation reaction of the first peptide and the second peptide can include phosphate buffer solution, Tris-hydrochloric acid buffer solution, citrate buffer solution, and the like, Among these, the phosphate buffer solution is preferable. The mixed solution of the buffer solution and the organic solvent used in the ligation reaction of the first peptide and the second peptide can include mixed solutions of the buffer solution described above and as organic solvent such as acetonitrile, DMF, or NMP. Among these, the mixed solution of phosphate buffer solution mid acetonitrile is preferable. The concentrations of the buffer solution and the mixed solution of buffer solution and organic solvent, and the mixing ratio of the mixed solution can be suitably adjusted depending on the addition amounts of the first peptide used and the second peptide used, and the kinds of the buffer solution and the organic solvent. For example, when the addition amounts of the first peptide and the second peptide are both 20 nmol, the concentration of the phosphate buffer solution used in the ligation reaction is preferably from 0.1 to 1.0 M, more preferably from 0.1 to 0.5 M, and further more preferably from 0.1 to 0.2 M. Moreover, when the addition amounts of the first peptide and the second peptide are both 20 nmol, the mixing ratio between the 0.1 M phosphate buffer solution and acetonitrile in the mixed solution used in the ligation reaction is preferably 1:1 of 0.1 M phosphate buffer solution and acetonitrile.

The ligation reaction is performed at a pH of preferably 5 to 9, more: preferably 6 to 8, and further more preferably 7±0.2. The temperature and the time of the ligation reaction can be suitably adjusted. For example, the reaction temperature can be set at room temperature

(10 to 40°C) and the reaction time can be set to a time during which the ligated product of the first peptide and the second peptide can be obtained in a predetermined amount.

In order to confirm the ligated product of the first peptide and the second peptide, a conventional method used for confirming a peptide can be used without any limitation, and for example, a method using an HPLC is preferable.

The yield of the ligated product of the first peptide and the second peptide is measured as follows:
The ligated product of the first peptide and the second peptide is subjected to an HPLC measurement by using a C18 column, and an aqueous acetonitrile solution containing 0.1%) (v/v) TPA as an eluent. The yield is calculated by dividing a peak area of a desired product aft 280 nm by the total of the peak area of desired product, a peak area of an unreacted C-terminal segment, and a peak area of a by-product derived from the C-terminal end.

The present invention will be explained in more detail by means, of Examples below, but the invention is not limited to the Examples.

### Example5

### Example 1

### (1) Synthesis of Compound 15 (Fmoc-Ser(CH₂-SCH₃)-OBu')

To Fmoc-Ser-OBu' (2.0 g 5.2 mmol) were added DMSO (10 ml, 140 mmol), AC₂O (6.6 ml, 70 mr.:101) and AcOH (10 ml, 180 mmol), and the mixture was caused to react u room temperature for two days. After vacuum concentration was carried out, distilled water was added thereto until precipitate was generated, and supernatant was removed therefrom. The precipitate was dissolved in ethyl acetate, which was washed with an aqueous saturated sodium bicarbonate solution and an aqueous saturated sodium chloride solution, and the resultant precipitate was dried with sodium sulfate. After the sodium sulfate was filtered off, vacuum concentration was carried out to give an oily material, and the oily material was purified through a silica gel column chromatography (silica gel 230 g, toluene: ethyl acetate = 9:1) to obtain compound 15 (1.8 g, 4.1 mmol, 79%).

R_{f} 0.48 (toluene: ethyl acetate = 5:1). ¹H-NMR (400 MHz, CDCl₃, TMS), δ 7.76 (d, 2H, J = 7.3 Hz, Ar), 7,61 (m, 2H, Ar), 7,4() (t, 2H, J = 7.3 Hz, Ar), 7.33 (t, 2H, J - 7.3 Hz, Ar), 5.63 (d, 1H, J - 8.3 Hz, No), 4.69 (d, 1H, J = 11,7 Hz, O-C*H*₂-S), 4.60 (d, 1H, J = 11.2 Hz, O-C*H*₂-S), 4.44 (m, 1H, αH), 4.39 (m, 2H. Fmoc C*H*₂), 4.24 (brt, 1H, J = 6.8 Hz, Fmoc C*H*), 3.99 (dd, 1H, J = 3.4,9.3 Hz, PH), 3.73 (dd, 1H, J = 2.9, 9.3 Hz, βH), 2.12 (s, 3H, S-C*H*₃). 1.46 (s, 9H, Bu^{t})_{.} HRFABMS: calcd for C₂₄H₂₉NO₅SNa 466.1664. Found: m/z 466.1632. [α]_{D}= +0.56^{a}(c = 1.0, CHCl₃).

### (2) Synthesis of Compound 16 (Fmoc-Ser(CH₂SSBu^{t})-OBu^{t})

Compound 15 described above (0.67g, 1.5 mmol) and DIEA (0.3i ml, 1.8 mmol) were dissolved in dichloromethane (5,0 ml), and then the resulting solution was cooled to 0°C, Dichloromethane (0,5 ml) in which SO₂Cl₂ (0,15 ml, 1,9 mmol) was dissolved was added thereto, and then the resulting solution was stirred at room temperature for one hour. After that, DMF (0.5 ml) in which potassium thiotosylate (0.51 g, 2.3 mmol) was dissolved was added thereto, and the mixture was stirred at room temperature tor 30 minutes. tBu-SH (0.34 ml, 4.7 mmol) was added thereto and the mixture was stirred at room temperature, The resulting mixture was diluted with dichloromethane, and an organic layer was washed with an aqueous saturated sodium bicarbonate solution and an aqueous sodium chloride solution, which was then dried with anhydrous calcium chloride. After the calcium chloride was filtered off, the solvent was distilled away under a reduced pressure, and the residue was purged through a gel column chromatography (toluene-ethyl acetate, 15:1) to obtain compound 16 (0.53 g, 1.0 mmol, 68%).

R_{f} 0.51(toluene: ethyl acetate, 9:1). ¹H-NMR (400 MHz, CDCl₃, TMS), δ 7.75 (d, 2H, J = 7.3 Hz, Ar), 7.63-7.60 (m, 2H, Ar), 7.39 (brt, 2H, J = 7.3 Hz, Ar), 7.30 (brt, 2H, J = 7.6 Hz, Ar), 5.65 (d, 1H, J = 8.3 Hz, NH), 4.85 (d, 1H, J = 11.2 Hz, O-C*H*₂-S), 4.75 (d, 1H, J = 11.2 Hz, O-C*H*2-S), 4.44 (m, 1H, αH), 4,23 (brt, 1H, J = 7.3 Hz. Fmoc C*H*), 4.05 (dd, 1H. J = 2.9, 9.3 Hz, β-H), 3.81 (dd, 1H, J = 2.7, 9.3 Hz, βH), 1.49 (s, 9H, Bu^{t}), 131 (s. 9H, Bu^{t}), HRFABMS: calcd for C₂₇H₃₅NO₅S₂Na 540.1854. Found: m/z 540.1744. [α]_{D}= +13.77^{a}(c = 1.3, CHCl₃)

### (3) Synthesis of Compound 10 (Fmoc-Ser(CH₂SSBu^{t})-OH)

Compound 16 described above (110 mg, 0.21 mmol) was dissolved in TFA (1.0 ml) containing 5% thioanisole, and the mixture was allowed to stand at room temperature for 30 minutes. After the solvent was distilled away under a reduced pressure, the obtained residue was purified through a silica gel column chromatography (chloroform-metharol, 15:1) to obtain compound 10 (60 mg, 0.13 mmol, 61%).

R_{f} 0.46 (chloroform-methanol, 9:1 containing 1% acetic acid). ¹H-NMR (400 MHz, CD₃SOCD₃), δ 7.88 (d, 2H, J = 7.8 Hz, Ar), 7.72 (d, 2H, J = 7.3 Hz Ar), 7.41 (brt, 2H, J = 7.5 Hz, Ar), 7.34 (brt, 2H, J = 7.5 Hz, Ar), 4.86 (d, 1H, J = 11.2 Hz, O-C*H*₂-S), 4.81 (d, 1H, J = 10.7 Hz, O-C*H*₂-S), 3.83-3.74 (m 2H, βH), 1.26 (s, 9H, Bu*^{t}*). HRFABMS: calcd for C₂₃H₂₃NO₅S₂Na 484.1228. Found: m/z, 484.1244. [α]_{D}= +26.9° (c = 0.75, CHCl₃).

### (4) Synthesis of Peptide 13(Ser(CH₂S-S-Bu^{t})-Asn-Ala-Thr(GalNAcDn)-Lys-Lys-Pro-Tyr-IIe-Leu-OH)

A Lys(Boc)-Lys(Boc)-Pro-Tyr(Bu*^{t}*)-Ile-Leu-CLEAR Acid resin was obtained from a starting material, Fmoc-Leu-CLEAR Acid resin (0.49 meq/g, 200 mg, 0.1 mmol) by the use of an automatic synthesizer according to a FastMoc method. To one third (0.033 mmol) of the amount of the resin obtained was added a solution of compound 12 or Fmoc-Thr(GalNAc)-OH (48 mg, 0.066 mmol), 0.45 M HBTU-HOBMt/DMF (138 µl, 0.063 mmol) and DIEA (23.0µl, 0.132 mmol) dissolved in NMP, and the mixture was caused to react at 50°C for one hour. Furthermore, the resulting product was condensed with Fmoc-Ala-OH (33 mg, 0.1 mmol) and then Fmoc-Asn(Trt)-OH (59.7 mg, 0.1 mmol) by using 1 M DCC (150 µl) and 1 M HOBt (150 µl). Finally, the resulting product was condensed with Fmoc-Ser(CH₂-S-S-Bu*^{t}*)-OH (31 mg, 0.066 mmol) by using 1 M DCC (99 µl) and 1 M HOBt (99 µl). After a half amount (0.017 mmol) of the obtained resin, in which the terminal Fmoc was removed, was dried, liquid (1 ml) of TFA: phenol: distilled water: thioanisole = 85:5:5:5 was added thereto, and the mixture was stirred for 30 minutes. The liquid was removed therefrom with nitrogen gas, to which diethyl ether was added to precipitate a peptide. This procedure was repeated three times, and the precipitates were dried under vacuum. The peptide was extracted with a 50% aqueous acetonitrile solution, and the resin was filtered off. Purification by using a reversed phase HPLC was made to obtain a desired peptide 13 (5.5 mg, 3.6 µmol, yield: 21%).

MALDI-TOFMS : found: m/z 1562.01 (M+H)⁺, calcd for m/z 1561.80 (M+H)⁺. Amino acid analysis: Asp_{0.96}Thr_{0.85}Ser_{0.90}Pro_{1.04}Ala_{1.00}Ile_{0.96}Leu_{1.06}Tyr_{0.98}Lys_{1.55}.

### (5) Synthesis of Peptide 14 (pGlu-Ser-Glu-Glu-Gly-Gly-SC₆H₄CH₂COOH)

After the Fmoc-CLEAR-Amide resin (0.46 meq/g, 440 mg, 0.2 mmol) was treated with 20% piperdine/NMP (5 nimutes x one time. 15 minutes x one time), and washed with NMP (one mimite x 5 times), a solution obtained by stirring the Fmoc-(Et)Cys(Trt)-OH (250 mg, 0.4 mmol), 1 M DCC/NMP (600 µl) and 1 M HOBt/NMP (600 µl) at room temperature for 30 minutes was added thereto and the mixture was shaken at 50°C for 1 hour. After the Fmoc was eliminated with piperidine (5 minutes x one time, and 15 minutes x one time), a NMP solution in which Fmoc-Gly-OH (300 mg, 1.0 mmol), HATU (380 mg, 1.0 mmol) and DIEA (350 µl, 2.0 mmol) were dissolved was added thereto, and the mixture was shaken at 50°C for 1 hour. This reaction was repeated once again. After a half amount (0.1 mmol) of the resin was taken out, the remaining resin was subjected to a treatment using an Applied Biosystems (ABI 433A) automatic synthesizer to extend the peptide chain in accordance with a FastMoc method, whereby an Ser(Bu*^{t}*)-Glu(OBu*^{t}*)-Glu(OBu*^{t}*)-Glu(OBu*^{t}*)-Gly-CLEAR amide resin was obtained. The resin was condensed with Boc-pGlu-OH (46 mg, 0.20 mmol) using 0.45 M HBTU, HOBt/DMF (420 µl, 0.19 mmol) and DIEA (70 µl, 0.40 mmol) to obtain Boc-pGlu-Ser(Bu*^{t}*)-Glu(OBu*^{t}*)-Gly-(OBu*^{t}*)Gly-CLEAR amide resin (330 mg). The whole amount of the resin was treated with Reagent K (TFA : phenol : distilled water : thioanisole : 3,6-dioxa-1,8-octanedithol = 825 : 5 : 5 : 5 : 2.5) (4.0 ml) for 2 hours, TFA was removed with nitrogen gas, to which diethyl ether was added to precipitate a peptide. This procedure was repeated three times, and the precipitate was dried under a reduced pressure. The precipitate was dissolved in a 30% aqueous acetonitrile solution (10 ml) containing 6 M urea, to which mercaptophenylacetic acid (0.2 ml) was added, and the mixture was caused to react overnight. After the resin was filtered, off, a crude peptide was purified through a reversed phase HPLC to obtain peptide 14 (4.3 mg, 5.8 µmol, 6%).

MALDI-TOF MS : found: m/z 739.137 (M+H)⁺, calcd for: m/z 739.225 (M+H)⁺. Amino acid analysis: Ser_{0.75}Glu_{2.63}Gly_{2.01}.

### (6) Synthesis of Peptide 17 (pGln-Ser-Glu-Glu-Gly-GLy-Ser-Asn-Ala-Thr(GalNAcBn)-Lys-Lys-Pro-Tyr-Tyr-Ile-Leu-OH) (see Table described above)

According to condition 1 in Table 1, peptide 13 and peptide 14 (20 mnol, each) were dissolved in a 0.1 M sodium phosphate buffer (pH 7.2, 4 µl) containing 6 M guanidine hydrochloride and 0.2 M MPAA, and the mixture was allowed to stand at room temperature overnight, According to condition 2 in Table 1, peptide 13 and peptide 14 (20 nmol, each) were dissolved in a 0.1 M sodium phosphate buffer (pH 7.0, 4 µl) containing 7.5 mM triscarboxyethyl phosphine (TCEP), and the mixture was allowed to stand at room temperature overnight. According to condition 3 in Table 1, peptide 13 and peptide 14 (20 nmol, each) were dissolved in 50% 0.1 M sodium phosphate buffer-acetonitrile (pH 7.0, 4 µl) containing 7.5 mM triscarboxyethyl phosphine (ICEP), and the mixture was allowed to stand overnight. According to condition 4 in Table 1, peptide 13 and peptide 14 (20 nmol, each) were dissolved in 50% 0.1 M sodium phosphate buffer-acetonitrile (pH 7.0, 4 µl) containing 7.5 mM triscarboxyetyl phosphine (TCEP), and DMF was added thereto after 10 minutes, and the mixture was allowed to stand overnight.

### (7) Synthesis of Compound 18 (Fmoc-Thr(CH₂SCH₂)-OBu^{t})

Fmoc-Thr-OBu^{t} (1.3 g, 3.3 mmol) was dissolved in DMSO (6.0 ml, 85 mmol), Ac₂O (4.0 ml, 42 mmol) and AcOH (6.0 ml, 0.10 mol), and the mixture was caused to react at room temperature for two days. After vacuum concentration, distilled water was added thereto until precipitate was generated, and a supernatant was removed therefrom. The precipitate was dissolved in ethyl acetate, the resulting solution was transferred to a separating funnel, and after that, was washed with an aqueous saturated sodium bicarbonate solution and an aqueous saturated sodium chloride solution. After the ethyl acetate layer was dried with sodium sulfate, followed by filtration, and vacuum concentration was made to obtain an oily material. The oily was purified through a silica gel column chromatography (silica gel 130 g, toluene: ethyl acetate = 9: 1) to obtain compound 18 (1.4 g, 3.0 mmol, 91%).

R_{f} 0.49 (toluene: ethyl acetate = 7: 1). ¹H-NMR (400 MHz, CDCl₃, TMS), d 7.75 (d, 2H, J=7.8 Hz, Ar), 7.62 (m, 2H, at), 7.39 (brt, H, J=7.3 Hz, Ar), 7.31 (t, 2H, J=7.3 Hz, Ar), 5.50 (d, 1H, J=9.8 Hz, NH), 4.65 (d, 1H, J=11.7 Hz, O-C*H*₂-S), 4.58 (d, 1H, J=11.7 Hz, O-C*H*₂-S). 4.35 (m, 1H, bH), 4.27 (dd, 1H, J= 2.0, 9.8 Hz, aH), 4.24 (brt, 1H, J=6,8 Hz, Fmoc CH), 2.13 (s, 3H,SCH₃), 1.49 (s, 9H, Bu*^{t}*), 1.23 (d, 3H, J=6.3 Hz, Thr CH₃), [a]_{D}= -19.1° (c 1.0 in Chloroform). MALDI-TOF MS: calcd for C₂₅H₃₁NNaO₅S 480.182. Found: m/z 480.054.

### (8) Synthesis of Compound 19 (Fmoc-Thr(CH₂SSBu^{t})-OBu^{t})

After compound 2 (0.50 g, 1.1 mmol) was azeotroped with toluene to dehydrate it and DCM (4.5 ml) was added thereto, DIEA (0/20 ml, 1.1 mmol) was added to the mixture to make it basic. After the mixture was cooled with ice, sulfuryl chloride (92 µl, 1.1 mmol) was added thereto, and the reaction was performed for 30 minutes, and then the temperature was returned to an ordinary temperature. Potassium thiotosylate (0.38 g, 1,7 mmol) dissolved in DMF (2,3 ml) was added to the mixture, and the reaction was performed for 30 minutes, to which t-butyl mercaptan (0.27 ml, 2.4 mmol) was added, and the reaction was performed for 30 minutes, Ethyl acetate was added to the reaction mixture, which was washed with a 1 M aqueous saturated HCl solution and then was dried with sodium sulfate. The resulting product was filtered and then, after vacuum concentration, purged through a silica gel column chromatography (90 g, hexane : ethyl acetate = 4: 1) to obtain compound 19 (0.48 g, 0.90 mmol, 82%).
R_{f} 0.38 (hexane : ethyl acetate = 4 : 1), ¹H-NMR (400 MHz, CDCl₃ TMS), d 7.75 (d, 2H, J=7.3 Hz, Ar), 7.62 (m, 2H, Ar), 7.39 (brt, H, J=7.6 Hz, Ar), 7.31 (brt, 2H, J=7.6 Hz, Ar), 5.49 (d, 1H, J=9.8 Hz, NH), 4.88 (d, 1H, J=11.2 Hz, O-C*H*₂-S), 4.75 (d, 1H, J=11,2 Hz, O-C*H*₂-S), 4,44 (m, 1H, bH), 4,28 (dd, 1H, J= 2.4,9.7 Hz, aH), 4.24 (brt, 1H, J=7.3 Hz, Fmoc CH), 1.51 (s, 9H, Bu*^{t}*), 1.33 (s, 9H, Bu*^{t}*), 1.27 (d, 3H, J=6,3 Hz, Thr CH₃). [a]_{D}= 19,1° (c 1.0 in Chloroform). MALDI-TOF MS, calcd for C₂₈H₃₇NNaO₅S₂ 554.201. Found: m/z 554.386.

### (9) Synthesis of Compound 11 (Fmoc-Thr(CH₂SSBu^{t})-OH)

Compound 5 (0.48 g, 0.90 mmol) was dissolved in 50% TFA- dichloromethane (5.0 ml) containing 5% thioanisole, and the reaction was performed for 30 minutes. After the reaction was finished, the reaction product was purified through a silica gel column chromatography (60 g, chloroform : methanol : acetic acid = 15 : 1 : 0,1), and then through a silica gel column chromatography (40 g, toluene : ethyl acetate : asetic acid = 2 : 1 : 0.1). After that, the product was purified through a gel filtration chromatography to obtain compound 11 (95 mg, 0.20 mmol, 22%).
R_{f} 0.39 (chloroform; methanol: acetic acid = 5 : 1 : 0.1). ¹H-NMR (400 MHz, CDCl₃, TMS), d 8.50 (brs, 1H, OH). 7.74 (d, 2H, J=8.0 Hz, Ar), 7,60 (m, 2H, Ar), 5.59 (m, 1H, NH), 5.10-4.71 (m, 2H, O-C*H*₂-S), 4,50-436 (m, 4H, aH, bH, Fmoc CH₂), 4.23 (t, 1H, J=6.8 Hz, Fmoc CM, 1.41-1.14 (m, 12H, Bu*^{t}*, Thr CH₃). [a]_{D}= 41.5° (c 1,0 in Chloroform). MALDI-TOF MS: calcd for C₂₄H₂₉NNaO₅S₂ 498.139. Found: m/z 498.084,

### Industrial Applicability

According to the method for producing a peptide of the present invention, a protein or glycoprotein having high stability can be solution-phase synthesized in a high yield, while the generation of by-products is restrained. The protein or glycoprotein obtained by the method for producing a peptide of the invention can be utilized in a production of pharmaceutical products. Furthermore, the glycoprotein produced by the present invention can be expected to show the same activity has that of a naturally occurring glycoprotein, such as an activity of bonding, to a specific cell receptor as one kind of nerve transmitter substances.

## Claims

1. A method for producing a peptide which comprises a step of causing a first peptide and a second peptide to react in the presence of a reducing agent to obtain a ligated product of the first peptide and the second peptide, wherein the first peptide contains, at the C-terminal end, an amino acid derivative having a thioester group, and the second peptide contains, at the N-terminal end, a serine or threonine derivative having a thiol auxiliary group.

2. The method for producing a peptide according to claim 1, wherein the thiol auxiliary group is a group containing a disulfide bond.

3. The method for producing a peptide according to claim 1 or 2, wherein the reducing agent is a phosphine compound.

4. The method for producing a peptide according to any one of claims 1 to 3, wherein the first peptide contains, at the C-terminal end, an amino acid derivative having a group represented by a formula: -CO-S-R
wherein R is an alkyl group having 1 to 12 carbon atoms which may be substituted by a carboxyl group, or an aryl group having 7 to 12 carbon atoms which may be substituted by a carboxyl group; and the second peptide contains, at the N-terminal end, a serine or threonine derivative having a group represented by a formula: -R₂-S-Y₁
wherein Y₁ is a protecting group of a thiol auxiliary group, and R₂ is a methylene group which may be substituted.

5. A compound represented by the following formula 1: 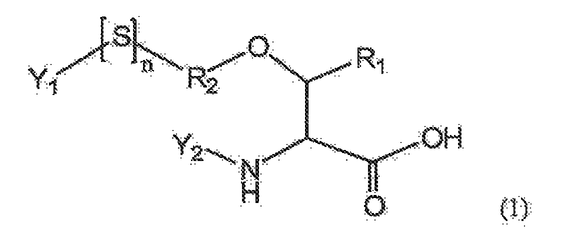 wherein Y₁ is a protecting group of a thiol auxiliary group, Y₂ is a protecting group of an amino group, R₁ is a hydrogen atom or a methyl group, R₂ is a methylene group which may be substituted, and n is an integer of 1 to 3.

6. The compound according to claim 5, wherein Y₁ is a t-butyl group, Y₂ is a 9-fluorenylmethoxycarbonyl group, R₁ is a hydrogen atom or a methyl group, R₂ is a methylene group, and n is 2.
